Europäisches Patentamt

European Patent Office

Office européen des brevets

(19)

(11) EP 0 499 541 B1

(12) FASCICULE DE BREVET EUROPEEN

(45) Date de publication et mention
de la délivrance du brevet:
14.08.1996 Bulletin 1996/33

(51) Int Cl.6: C07K 1/14, C07K 1/30

(21) Numéro de dépôt: 92400381.7

(22) Date de dépôt: 13.02.1992

(54) **Procédé de purification d'une protéine fortement glycosylée**

Verfahren zur Reinigung eines hochglykosylierten Proteins

Process for the purification of a highly glycosylated protein

(84) Etats contractants désignés:
AT BE CH DE DK ES FR GB IT LI NL SE

(30) Priorité: 15.02.1991 FR 9101830

(43) Date de publication de la demande:
19.08.1992 Bulletin 1992/34

(73) Titulaire: TRANSGENE S.A.
67000 Strasbourg (FR)

(72) Inventeur: Kolbe, Hanno
F-67400 Illkirch (FR)

(74) Mandataire: Warcoin, Jacques et al
Cabinet Régimbeau,
26, avenue Kléber
75116 Paris (FR)

(56) Documents cités:
EP-A- 0 277 043            EP-A- 0 321 606

• FEBS LETTERS, vol. 80, no. 2, 1977, Amsterdam
(NL); LEBRETON et al., pp. 351-354
• CHEMICAL ABSTRACTS, vol. 96, 1982,
Columbus, OH (US); SULKOWSKI et al., p. 536,
AN 102224a
• PROTEIN PURIFICATION, MICRO TO MACRO,
1987, Alan R. Liss Inc., New York, NY (US); E.
SULKOWSKI, pp. 149-162

## EP 0 499 541 B1

**Description**

La présente invention concerne un procédé de purification d'une protéine fortement glycosylée.

Par définition, une protéine est essentiellement constituée d'une chaîne d'acides aminés. De plus, dans un grand nombre de cas, cette chaîne porte en quantité plus ou moins importante des groupements glucidiques de taille variable. Les protéines portant de tels groupements sont appelées protéines glycosylées.

D'une manière générale, il est connu que des métaux tels que le zinc, le cuivre, le cobalt, le calcium ou le nickel peuvent se complexer à des composés de nature protéique. Ainsi ces métaux sont couramment utilisés dans des procédés de purification de protéines ; soit en tant qu'agent de couplage, par exemple lors d'une chromatographie d'affinité; soit pour précipiter des protéines en milieu liquide.

De manière surprenante, il a maintenant été trouvé que des protéines fortement glycosylées ne sont pas capables de se complexer aux métaux mentionnés ci-dessus.

En conséquence, l'invention propose un procédé pour purifier une protéine fortement glycosylée à partir d'une préparation brute dérivée d'une culture de cellules eucaryotes, ledit procédé comprenant l'acte (i) d'ajouter à ladite préparation un ion métallique bivalent en quantité suffisante de manière à former un mélange qui précipite et (ii), après précipitation, de récolter ladite protéine dans le surnageant du mélange.

Par protéine fortement glycosylée, on entend une protéine ayant un taux de glycosylation d'au moins 20%, de préférence d'au moins 30%, de manière tout à fait préférée, d'au moins 40%. Le taux de glycosylation est le rapport entre la masse de la glycosylation et la masse totale de la protéine, exprimé en pourcentage. Ce taux de glycosylation peut être déterminé en comparant la masse d'une protéine glycosylée à celle de sa forme non-glycosylée obtenue après traitement à l'aide d'une endoglucosidase (endoglucosidase F ou H vendue par exemple, par Boehringer) selon les conditions indiquées par le fournisseur. Les masses relatives des formes glycosylées et non-glycosylées sont déterminées après migration sur gel de SDS-PAGE.

De manière alternative, si on connait la séquence en acide aminé de la protéine glycosylée on peut calculer la masse de la chaine peptidique. La quantification des sucres peut être effectuée en parallèle après hydrazinolyse de la protéine glycosylée.

La fétuine, les glycoprotéines gp120 et gp160 du virus HIV, les mucines, l'interleukine-2 humaine, les protéines "glycophorine like" et l'alpha-1-glycoprotéine acide présente dans le sérum, sont par exemple des protéines présentant naturellement un fort taux de glycosylation. Aux fins de la présente invention, la protéine que l'on souhaite purifier peut avoir un profil de glycosylation homogène (glycosylation répartie sur toute la chaine peptidique) ou hétérogène (glycosylation localisée en certains endroits de la chaine peptidique). De préférence, la glycosylation est homogène.

La préparation brute à partir de laquelle on souhaite purifier une protéine fortement glycosylée dérive d'une culture de cellules eucaryotes capable de synthétiser cette protéine. La composition du milieu de culture n'est pas critique et peut contenir n'importe quel constituant approprié. Les différentes méthodes qui permettent d'obtenir la synthèse d'une protéine par voie recombinante sont connues ; l'élément de base à introduire dans une cellule étant la cassette d'expression destinée à la synthèse de cette protéine. Avantageusement, une cassette d'expression comporte un fragment d'ADN codant un précurseur de la protéine fortement glycosylée (peptide signal + protéine mature) placé sous le contrôle d'éléments nécessaires à son expression.

Dans le cadre de la présente invention, une protéine fortement glycosylée est avantageusement purifiée à partir d'un extrait acellulaire dérivé d'une culture de cellules eucaryotes, de préférence d'une culture de levure ou cellules de mammifère. Cet extrait acellulaire peut être un surnageant de culture de cellules dans les cas où soit la protéine est sécrétée dans le milieu de culture, soit les cellules sont, en cours de culture, lysées par un virus. De manière alternative, l'extrait acellulaire peut être aussi obtenu à partir d'une suspension cellulaire soumise à un traitement lytique de type chimique, mécanique ou enzymatique et dont l'élément liquide est ensuite séparé des débris cellulaires.

Pour usage dans le cadre de la présente invention, un ion métallique bivalent est avantageusement sélectionné parmi le $Zn^{2+}$, $Cu^{2+}$, $Co^{2+}$, $Ni^{2+}$ et $Ca^{2+}$. De préférence, cet ion métallique est ajouté sous forme saline. A titre d'exemple, on cite le $ZnCl_2$, le $CuSO_4$, le $CoSO_4$, le $NiSO_4$ et le $CaCl_2$.

D'une manière générale, la quantité d'ion métallique qu'il convient d'ajouter à la préparation brute dépend de la nature même de l'ion métallique et des constituants que l'on souhaite éliminer. L'homme du métier saura bien évidemment adapter la concentration finale optimale en fonction des données spécifiques. Toutefois, on indique que les contaminants présents dans la préparation brute peuvent précipiter dès que l'on ajoute l'ion métallique à une concentration finale d'au moins 10 mM, avantageusement d'au moins 40 mM, de préférence d'au moins 60 mM, de manière tout à fait préférée d'au moins 80 mM. Bien qu'il soit possible d'ajouter l'ion métallique en quantité, il n'est généralement pas nécessaire de dépasser une concentration finale d'environ 100 à 120 mM.

Avantageusement, le procédé selon l'invention est effectué à un pH acide ou proche de la neutralité, en général de 7,5 ou inférieur à 7,5. De préférence, le procédé est effectué à un pH légèrement acide compris entre 6 et 7.

Après précipitation, le surnageant est avantageusement récolté par centritugation du mélange et décantation successive.

Un tel procédé permet entre autre d'éliminer, de manière simple et efficace, les virions qui peuvent se trouver présents dans la préparation brute. Si nécessaire, la purification de la protéine fortement glycosylée présente dans le surnageant de précipitation peut être par la suite poursuivie de manière plus fine selon des méthodes variées.

Selon un aspect particulier, l'invention propose un procédé de purification de la gp120 du virus HIV ou d'un variant soluble de la gp160 du virus HIV. Certains variants de la gp160 sont décrits, par exemple, dans la demande de brevet EPA 245 136. La gp120 ou un variant soluble de la gp160 peut être notamment produit par les techniques de l'ADN recombinant. Un vecteur destiné à cette production est par exemple un plasmide ou un virus de la vaccine dans le génome duquel est inséré un fragment d'ADN codant pour la gp120 ou un variant soluble de la gp160, celui-ci étant placé sous le contrôle de régions appropriées permettant son expression (transcription et traduction).

Enfin, de manière alternative, l'invention concerne un procédé pour purifier ou concentrer un poxvirus à partir d'une préparation, par exemple surnageant d'un lysat cellulaire obtenu par infection d'une culture de cellules infectées par ledit poxvirus, le dit procédé comprenant l'acte (i) d'ajouter à ladite préparation un ion métallique bivalent en quantité suffisante de manière à former un mélange qui précipite et (ii), après précipitation, de récolter ledit poxvirus sous forme d'un précipité.

Exemple 1 :

A. Production de la gp120 de HIV1-BRU en culture de cellules infectées par un virus de la vaccine recombinant :

Des cellules BHK-21 sont cultivées dans un milieu GMEM (Gibco) supplémenté avec 10% de sérum de veau foetal (SVF). Lorsque les cellules sont à confluence ($5,8 \times 10^6$ cellules/ml), le milieu de culture est enlevé et le tapis cellulaire est lavé 2 fois avec 50 ml de PBS (Dubelcco's phosphate buffer salt ; Seromed). Puis du milieu GMEM frais sans SVF est rajouté. On ajoute alors le virus de la vaccine VVTG1132, décrit dans la demande de brevet EPA 245 136, à une infectivité de $10^{-1}$ ufp (unité formant plages), et l'infection est poursuivie pendant 72 h. Le lysat est alors centrifugé 20 min. à 10 000 g pour éliminer les débris cellulaires et on récupère le surnageant contenant entre autre la gp120 et des virions.

B. Purification de la gp120 :

A 20 ml de surnageant de culture obtenu comme précédemment décrit, on ajoute 1,3 ml d'une solution de chlorure de zinc ($ZnCl_2$) à 1 M pour avoir une concentration finale en $ZnCl_2$ de 60 mM. Le pH de ce mélange est d'environ 6,8. Le mélange est laissé 1 h dans de la glace puis le surnageant de précipitation est récupéré après centrifugation à 3000 t/min. pendant 20 min. dans une centrifugeuse Minifuge RF Heraeus. Celui-ci contient la quantité initiale de gp120 trouvée dans le surnageant de culture mais ne contient plus entre autre, de virion. Pour analyse, on effectue une quantification des protéines totales et de la gp120 ainsi qu'une titration de virus de la vaccine par les méthodes décrites ci-dessous. Ces quantifications sont faites en parallèle sur le surnageant de culture brut non traité au $ZnCl_2$.

La quantification des protéines totales s'effectue comme suit selon la méthode du Biuret décrite par Kadenbach B, Biochem. Z. (1966) 344 : 49-75 : 3 ml de surnageant obtenu après précipitation par le $ZnCl_2$ sont traités avec 300 µl d'acide trichloracétique (TCA) 3 M. Après 1 h à 20 °C les protéines sont précipitées et la solution est centrifugée 20 min. à 3000 tours par minute dans une centrifugeuse Minifuge RF Heraus. Le surnageant est éliminé et le culot est repris dans 1 ml de solution Biuret (12 mM de sulfate de cuivre ($CuSO_4$, $5H_2O$), 31,89 mM tartrate de sodium potassium (KNaTartrate), 200 mM de soude (NaOH) et 30,12 mM de iodure de potassium (IK)). Après 15 min. les protéines sont dissoutes dans la solution de Biuret et on rajoute 1 ml d'eau Milli-Q. La densité optique (DO) à 546 nm est mesurée dans des cuvettes de 1 cm contre l'air. On mesure également la DO d'un témoin d'absorption dans lequel seules la solution de Biuret et l'eau ont été rajoutées. La valeur de la DO est notée DO.E pour les échantillons ou DO.T pour le témoin d'absorption. 50 à 100 mg de cyanure de potassium (KCN) sont alors rajoutés dans les cuvettes et mélangés dans la solution à l'aide d'une spatule. Après 2 à 5 min. la DO à 546 nm est mesurée et sa valeur est notée DO.E/KCN pour les échantillons et DO.T/KCN pour le témoin d'absorption. La quantité totale de protéines dans les échantillons (Q) se calcule alors par la formule suivante :

$$Q = 7 \times (DO.E - DO.E/KCN) - (DO.T - DO.T/KCN)$$

La quantification de la gp120 s'effectue par test ELISA en utilisant deux anticorps monoclonaux anti-gp120 comme premier et deuxième anticorps, le second étant biotinilé. La marge d'incertitude associée à une telle méthode d'estimation est de l'ordre de ± 20%

La titration du virus s'effectue sur des cellules BHK-21. 2 ml de milieu GMEM contenant 10% de SVF sont ensemencés avec $10^6$ cellules BHK-21. La culture se fait à 37°C dans des boites de 35x10 mm. Après 24 h le milieu est enlevé et 200 µl d'une dilution du virus de la vaccine dans du PBS sont rajoutés sur les cellules. L'adsorption se fait pendant 1 h à 37°C, puis on ajoute 2 ml de milieu GMEM contenant 5 % de SVF et l'infection est poursuivie pendant 24 h à 37°C. Le milieu est alors retiré et on colore les cellules avec 1 ml de rouge neutre (Gibco). On compte les plages de lyse restées blanches.

Les résultats sont présentés dans le tableau 1 ci-dessous.

Tableau 1

| Echantillons | Concentration des protéines totales (µg/ml) | Concentration de la gp120 (µg/ml) | Titration du virus de la vaccine (ufp/ml) |
|---|---|---|---|
| Surnageant de culture | 257 | 0,202 | $10^6$ |
| Surnageant après précipitation au $ZnCl_2$ | 61,3 | 0,206 | 0 |

Ces résultats montrent que la protéine gp120 n'est pas précipitée par le $ZnCl_2$ alors que la majorité des protéines contaminantes le sont. De même le virus de la vaccine est éliminé dans le culot de précipitation.

Grâce à ce procédé, il est donc possible d'éliminer en une seule étape les virions ainsi que plus de 50% des protéines contaminantes.

Exemples 2 à 6:

Un extrait acellulaire contenant la gp120 est obtenu comme décrit dans l'Exemple 1A. De même, la gp120 est purifiée selon des procédés similaires à celui présenté à l'Exemple 1B dans lequel seule la concentration finale en $ZnCl_2$ peut varier de 5 mM à 80 mM. Les résultats de ces expériences sont rapportés dans le tableau 2 ci-dessous.

Tableau 2

| | Echantillons | Concentration en protéines totales (µg/ml) | Concentration en variant gp120 (µg/ml) | Titration du virus de la vaccine (ufp/ml) |
|---|---|---|---|---|
| Contrôle | Surnageant de culture | 257 | 0,202 | $10^6$ |
| Ex 2 | Surnageant après précipitation avec 5 mM de $ZnCl_2$ | 87 | 0,161 | $2x10^4$ |
| Ex 3 | Surnageant après précipitation avec 10 mM de $ZnCl_2$ | 74 | 0,182 | $10^3$ |
| Ex 4 | Surnageant après précipitation avec 20 mM de $ZnCl_2$ | 72 | 0,184 | $5x10^2$ |
| Ex 5 | Surnageant après précipitation avec 40 mM de $ZnCl_2$ | 63 | 0,248 | 50 |
| Ex 6 | Surnageant après précipitation avec 80 mM de $ZnCl_2$ | 69 | 0,254 | < 50 |

Ces expériences montrent que les protéines contaminantes et le virus de la vaccine commencent à précipiter dès 5 mM de $ZnCl_2$. On considère que la gp120 ne précipite pas compte tenu de la marge d'incertitude de ± 20% associée à la méthode d'estimation.

Exemple 7:

A 20 ml de surnageant de culture contenant la gp120 obtenu comme précédemment décrit dans l'exemple 1A, on ajoute 1,3 ml d'une solution de chlorure de calcium ($CaCl_2$) à 1 M pour avoir une concentration finale en $CaCl_2$ de 60 mM. Le pH de ce mélange est d'environ 6,8. Le mélange est laissé 1 h dans de la glace puis le surnageant de précipitation est récupéré après centrifugation à 3000 t/min. pendant 20 min. dans une centrifugeuse Minifuge RF Heraeus.

Celui-ci contient la quantité initiale de gp120 trouvée dans le surnageant de culture mais ne contient plus entre autre de virion. Pour analyse, on effectue une quantification des protéines totales et de la gp120 ainsi qu'une titration de virus de la vaccine par les méthodes décrites dans l'exemple 1B. Ces quantifications sont faites en parallèle sur le surnageant de culture brut non traité au $CaCl_2$.

Les résultats de ces expériences sont rapportés dans le tableau 3 ci-dessous.

Tableau 3

| Echantillons | Concentration des protéines totales (µg/ml) | Concentration de la gp120 (µg/ml) | Titration du virus de la vaccine (ufp/ml) |
|---|---|---|---|
| Surnageant de culture | 218 | 0,360 | 1,5 $10^5$ |
| Surnageant après précipitation au $CaCl_2$ | 139 | 0,374 | <100 |

Ces résultats montrent que le $CaCl_2$ tout comme le $ZnCl_2$ précipite la majorité du virus de la vaccine alors que la gp120 reste en solution.

Exemples 8 à 10 :

Un extrait acellulaire contenant la gp120 est obtenu comme décrit dans l'Exemple 1A. De même, la gp120 est purifiée selon des procédés similaires à celui présenté à l'Exemple 7 dans lequel seule la concentration finale en $CaCl_2$ peut varier de 20 mM à 80 mM. Les résultats de ces expériences sont rapportés dans le tableau 4 ci-dessous.

Tableau 4

| | Echantillons | Concentration en protéines totales (µg/ml) | Concentration en variant gp120 (µg/ml) | Titration du virus de la vaccine (ufp/ml) |
|---|---|---|---|---|
| Contrôle | Surnageant de cellules | 218 | 0,280 | 1,5x$10^5$ |
| Ex 8 | Surnageant après précipitation avec 20 mM de $CaCl_2$ | 163 | 0,282 | 7x$10^3$ |
| Ex 9 | Surnageant après précipitation avec 40 mM de $CaCl_2$ | 149 | 0,330 | < 100 |
| Ex 10 | Surnageant après précipitation avec 80 mM de $CaCl_2$ | 127 | 0,346 | < 100 |

Ces expériences montrent que les protéines contaminantes et le virus de la vaccine commencent à précipiter dès 10 mM de $CaCl_2$.

Exemple 11:

Un extrait acellulaire contenant un variant soluble de la gp160 de HIV1-BRU, est obtenu après infection de cellules BHK-21 par le virus de la vaccine VVTG1163 décrit dans la demande de brevet EPA 245 136, selon la méthode décrite dans l'exemple 1A.

A 20 ml de surnageant de culture contenant le variant soluble de la gp160 obtenu comme précédemment décrit, on ajoute 880 µl d'une solution de $ZnCl_2$ à 1 M pour avoir une concentration finale en $ZnCl_2$ de 40 mM. Le mélange est laissé 1 h dans de la glace puis le surnageant de précipitation est récupéré après centrifugation à 3000 t/min. pendant 20 min. dans une centrifugeuse Minifuge RF Heraeus. La quantité du variant gp160 présente dans le surnageant après précipitation au $ZnCl_2$ est comparable à celle trouvée dans le surnageant de culture ; par contre la majorité des protéines contaminantes sont éliminées ainsi que la totalité des virions.

Exemple 12 :

Des cellules CHO sont transformées par le plasmide pGT3554. Ce plasmide est obtenu par insertion dans le plasmide pTG384 (décrit dans la demande de brevet FR 2 600 334) préalablement digéré par HindIII et traité par le fragment Klenow de l'ADN polymérase I, d'un fragment PstI traité par la polymérase Klenow codant pour un variant soluble de la gp 160 HIV-1 BRU, qui dérive un vecteur vaccine VVTG 1163 (décrit dans la demande de brevet WO 87/6260). Le fragment ainsi clone est placé sous le contrôle du promoteur majeur tardif de l'adénovirus 2.

Environ $10^6$ cellules sont ensemencées en milieu MEM $\alpha$ 2000 (Gibco) complementé en sérum de veau foetal dialysé 10 %, glucose 2 g/l, hypoxanthine 15 mg/l, thymidine 10 mg/l, xanthine 250 mg/l, aminoptérine 0.2 mg/l, acide mycophénolique 25 mg/l et glutamine 2 mM ; puis cultivées 3 jours à 37 °C en atmosphère chargée en $CO_2$ 5%. Puis le milieu est remplacé chaque jour pendant 2 jours par du milieu frais MEM $\alpha$ 2000 complémenté en sérum de veau foetal (inactivé à 56 °C) 10 %, glucose 2 g/l, hypoxanthine 15 mg/l, thymidine 10 mg/l, xanthine 250 mg/l, aminoptérine 0.2 mg/l, acide mycophénolique 25 mg/l et glutamine 2 mM.

Le surnageant de culture est ensuite récuperé et purifie tel que décrit dans l'Exemple 11. La quantité du variant gp 160 présente dans le surnageant après précipitation au $ZnCl_2$ est comparable à celle trouvée dans le surnageant de culture ; par contre, la quantité de protéines totales a fortement diminué, indiquant ainsi qu'une bonne partie des protéines contaminantes est éliminée.

**Revendications**

1. Un procédé pour purifier une protéine fortement glycosylée à partir une préparation brute dérivé une culture de cellules eucaryotes, qui comprend l'acte (i) d'ajouter à ladite préparation un ion métallique bivalent en quantité suffisante de manière à former un mélange qui précipite et (ii), après précipitation, de récolter ladite protéine dans le surnageant du mélange.

2. Un procédé selon la revendication 1, pour purifier une protéine fortement glycosylée à partir d'une préparation brute qui est un extrait acellulaire dérivé d'une culture de cellules, qui comprend l'acte (i) d'ajouter à ladite préparation un ion métallique bivalent en quantité suffisante de manière à former un mélange qui précipite et (ii), après précipitation, de récolter ladite protéine dans le surnageant du mélange.

3. Un procédé selon la revendication 2, pour purifier une protéine fortement glycosylée à partir d'une préparation brute qui est un extrait acellulaire dérivé d'une culture de cellules infectées par un virus de la vaccine exprimant ladite protéine fortement glycosylée, qui comprend l'acte (i) d'ajouter à ladite préparation un ion métallique bivalent en quantité suffisante de manière à former un mélange qui précipite et (ii), après précipitation, de récolter ladite protéine dans le surnageant du mélange.

4. Un procédé selon l'une quelconque des revendications 1 à 3, pour purifier à partir d'une préparation brute, une protéine dont le taux de glycosylation est d'au moins 40%, qui comprend l'acte (i) d'ajouter à ladite préparation un ion métallique bivalent en quantité suffisante de manière à former un mélange qui précipite et (ii), après précipitation, de récolter ladite protéine dans de surnageant du mélange.

5. Un procédé selon n'importe laquelle des revendications 1 à 4, pour purifier une protéine fortement glycosylée à partir d'une préparation brute, qui comprend l'acte d'ajouter à ladite préparation un ion métallique bivalent pour former un mélange dans lequel l'ion métallique a une concentration finale d'au moins 10 mM.

6. Un procédé selon la revendication 5, pour purifier une protéine fortement glycosylée à partir d'une préparation brute, qui comprend l'acte d'ajouter à ladite préparation un ion métallique bivalent pour former un mélange dans lequel l'ion métallique a une concentration finale d'au moins 20 mM.

7. Un procédé selon n'importe laquelle des revendications 1 à 6, pour purifier une protéine fortement glycosylée à partir d'une préparation brute, qui comprend l'acte d'ajouter à ladite préparation un ion métallique bivalent sélectionné parmi le zinc, le cuivre, le cobalt ou le nickel.

8. Un procédé selon n'importe laquelle des revendications 1 à 7, pour purifier une protéine fortement glycosylée à partir d'une préparation brute, qui comprend l'acte d'ajouter à ladite préparation un ion métallique bivalent sous forme saline.

9. Un procédé selon n'importe laquelle des revendications 1 à 8, dans lequel la protéine fortement glycosylée est la protéine gp120 du virus HIV.

10. Un procédé selon n'importe laquelle des revendications 1 à 8, dans lequel la protéine fortement glycosylée est un variant soluble de la protéine gp160 du virus HIV.

11. Un procédé pour purifier ou concentrer un poxvirus à partir d'une préparation, qui comprend l'acte (i) d'ajouter à ladite préparation un ion métallique bivalent en quantité suffisante de manière à former un mélange qui précipite et (ii), après précipitation, de récolter ledit poxvirus sous forme précipitée.

**Patentansprüche**

1. Verfahren zur Reinigung eines stark glycosylierten Proteins, bei dem man von einem aus einer Kultur von Eukaryontenzellen stammenden rohen Präparat ausgeht, das umfaßt

(i) die Zugabe eines bivalenten Metallions zu dem genannten Präparat in ausreichender Menge zur Bildung einer Mischung, die ausfällt, und
(ii) die Gewinnung (Abtrennung) des genannten Proteins aus dem Flüssigkeitsüberstand der Mischung nach der Ausfällung.

2. Verfahren nach Anspruch 1 zur Reinigung eines stark glycosylierten Proteins, bei dem man von einem rohen Präparat ausgeht, bei dem es sich um einen aus einer Zellkultur stammenden zellfreien Extrakt handelt, das umfaßt (i) die Zugabe eines bivalenten Metallions zu dem genannten Präparat in ausreichender Menge zur Bildung einer Mischung, die ausfällt, und (ii) die Gewinnung (Abtrennung) des genannten Proteins aus dem Flüssigkeitsüberstand der Mischung nach der Ausfällung.

3. Verfahren nach Anspruch 2 zur Reinigung eines stark glycosylierten Proteins, bei dem man von einem rohen Präparat ausgeht, bei dem es sich um einen zellfreien Extrakt handelt, der aus einer Kultur von Zellen stammt, die durch ein Vaccine-Virus infiziert worden sind, welches das genannte stark glycosylierte Protein exprimiert, das umfaßt

(i) die Zugabe eines bivalenten Metallions zu dem genannten Präparat in ausreichender Menge zur Bildung einer Mischung, die ausfällt, und
(ii) die Gewinnung (Abtrennung) des genannten Proteins aus dem Flüssigkeitsüberstand der Mischung nach der Ausfällung.

4. Reinigungsverfahren nach einem der Ansprüche 1 bis 3, bei dem man von einem rohen Präparat, einem Protein mit einem Glycosylierungs-Grad von mindestens 40 %, ausgeht, das umfaßt

(i) die Zugabe eines bivalenten Metallions zu dem genannten Präparat in ausreichender Menge zur Bildung einer Mischung, die ausfällt, und
(ii) die Gewinnung (Abtrennung) des genannten Proteins aus dem Flüssigkeitsüberstand der Mischung nach der Ausfällung.

5. Verfahren nach einem der Ansprüche 1 bis 4 zur Reinigung eines stark glycosylierten Proteins, bei dem man von einem rohen Präparat ausgeht, das umfaßt die Zugabe eines bivalenten Metallions zu dem genannten Präparat zur Bildung einer Mischung, in der das Metallion eine End-Konzentration von mindestens 10 mM hat.

6. Verfahren nach Anspruch 5 zur Reinigung eines stark glycosylierten Proteins, bei dem man von einem rohen Präparat ausgeht, das umfaßt die Zugabe eines bivalenten Metallions zu dem genannten Präparat zur Bildung einer Mischung, in der das Metallion eine End-Konzentration von mindestens 20 mM hat.

7. Verfahren nach einem der Ansprüche 1 bis 6 zur Reinigung eines stark glycosylierten Proteins, bei dem man von einem rohen Präparat ausgeht, das die Zugabe eines bivalenten Metallions, ausgewählt aus der Gruppe Zink, Kupfer, Kobalt und Nickel, zu dem genannten Präparat umfaßt.

8. Verfahren nach einem der Ansprüche 1 bis 7 zur Reinigung eines stark glycosylierten Proteins, bei dem man von einem rohen Präparat ausgeht, das die Zugabe eines bivalenten Metallions in Form einer Salzlösung zu dem genannten Präparat umfaßt.

**9.** Verfahren nach einem der Ansprüchen 1 bis 8, bei dem das stark glycosylierte Protein das Protein gp120 des HIV-Virus ist.

**10.** Verfahren nach einem der Ansprüche 1 bis 8, bei dem das stark glycosylierte Protein eine lösliche Variante des Proteins gp160 des HIV-Virus ist.

**11.** Verfahren zur Reinigung oder Konzentrierung eines Pockenvirus, bei dem man von einem Präparat ausgeht, das umfaßt

(i) die Zugabe eines bivalenten Metallions zu dem genannten Präparat in einer ausreichender Menge zur Bildung einer Mischung, die ausfällt, und
(ii) die Gewinnung (Abtrennung) des genannten Pockenvirus in der ausgefallenen Form nach der Ausfällung.

## Claims

**1.** A process for purifying a highly glycosylated protein from a crude preparation derived from a culture of eukaryotic cells, which comprises the action (i) of adding to the said preparation a divalent metal ion in a sufficient amount in order to form a mixture which precipitates and (ii) after precipitation, of harvesting the said protein from the mixture supernatant.

**2.** A process according to Claim 1, for purifying a highly glycosylated protein from a crude preparation which is an acellular extract derived from a cell culture, which comprises the action (i) of adding to the said preparation a divalent metal ion in a sufficient amount in order to form a mixture which precipitates and (ii) after precipitation, of harvesting the said protein from the mixture supernatant.

**3.** A process according to Claim 2, for purifying a highly glycosylated protein from a crude preparation which is an acellular extract derived from a culture of cells infected with a vaccinia virus expressing the said highly glycosylated protein, which comprises the action (i) of adding to the said preparation a divalent metal ion in a sufficient amount in order to form a mixture which precipitates and (ii) after precipitation, of harvesting the said protein from the mixture supernatant.

**4.** A process according to any one of Claims 1 to 3, for purifying from a crude preparation a protein whose glycosylation level is not less than 40%, which comprises the action (i) of adding to the said preparation a divalent metal ion in a sufficient amount in order to form a mixture which precipitates and (ii) after precipitation, of harvesting the said protein from the mixture supernatant.

**5.** A process according to any one of Claims 1 to 4, for purifying a highly glycosylated protein from a crude preparation, which comprises the action of adding to the said preparation a divalent metal ion in order to form a mixture in which the metal ion is at a final concentration of not less than 10 mM.

**6.** A process according to Claim 5, for purifying a highly glycosylated protein from a crude preparation, which comprises the action of adding to the said preparation a divalent metal ion in order to form a mixture in which the metal ion is at a final concentration of not less than 20 mM.

**7.** A process according to any one of Claims 1 to 6, for purifying a highly glycosylated protein from a crude preparation, which comprises the action of adding to the said preparation a divalent metal ion selected from zinc, copper, cobalt or nickel.

**8.** A process according to any one of Claims 1 to 7, for purifying a highly glycosylated protein from a crude preparation, which comprises the action of adding to the said preparation a divalent metal ion in the form of a salt.

**9.** A process according to any one of Claims 1 to 8, in which the highly glycosylated protein is the protein gp120 from the HIV virus.

10. A process according to any one of Claims 1 to 8, in which the highly glycosylated protein is a soluble variant of the protein gp160 from the HIV virus.

11. A process for purifying or concentrating a poxvirus from a preparation, which comprises the action (i) of adding to the said preparation a divalent metal ion in a sufficient amount in order to form a mixture which precipitates and (ii) after precipitation, of harvesting the said poxvirus in the form of a precipitate.